# EUROPEAN PATENT APPLICATION

(11) **EP 1 698 685 A1**
(43) Date of publication of application: **06.09.2006**
(21) Application number: 04807408.2
(22) Date of filing: 21.12.2004
(51) Int. Cl.: C11C 3/00, A61K 31/202, A61K 31/683, A23D 9/00, A23L 1/30, A23J 7/00, A61P 25/20, A61P 25/28

(54) **FAT COMPOSITION**

(30) Priority: 22.12.2003 JP 2003424791
(71) Applicant: WAKUNAGA PHARMACEUTICAL CO., LTD., Osaka-shi, Osaka 532-0003 (JP)
(72) Inventor: MORIGUCHI, Toru, WAKUNAGA PHARMACEUTICAL CO., LTD., AKITAKATA-SHI, Hiroshima 7391195 (JP); SASAKI, Tetsuyuki, WAKUNAGA PHARMACEUTICAL CO.,LTD, AKITAKATA-SHI, Hiroshima 7391195 (JP); HARAUMA, Akiko, WAKUNAGA PHARMACEUTICAL CO., LTD, AKITAKATA-SHI, Hiroshima 7391195 (JP)
(74) Representative: Hartz, Nikolai
(86) International application number: PCT/JP2004/019051
(87) International publication number: WO 2005/061684

(57) **Abstract**

Provided is a composition permitting high systemic absorption of n-3 polyunsaturated fatty acids and high transfer of them to tissues and capable of fully exhibiting pharmacological effects which the fatty acids have.

A fat composition containing one or more phospholipids having, as a constituent fatty acid, an n-3 polyunsaturated fatty acid selected from docosahexaenoic acid, docosapentaenoic acid and eicosapentaenoic acid; and α-linolenic acid and/or a fat containing α-linolenic acid.

## Description

### TECHNICAL FIELD

The present invention relates to a fat composition comprising a phospholipid having, as a constituent fatty acid, an n-3 polyunsaturated fatty acid such as docosahexaenoic acid; and an α-linolenic acid and/or a fat containing an α-linolenic acid.

### BACKGROUND ART

Docosahexaenoic acid (DHA), docosapentaenoic acid (DPA) and eicosapentaenoic acid (EPA) are contained in large amounts in fish oils and marine mammal oils such as seal oil. They are all n-3 polyunsaturated fatty acids.
It is reported that they have, as common pharmacological effects, a neutral fat lowering effect and a blood platelet aggregation inhibitory effect. It is also reported that they have the other pharmacological effects, for example, docosahexaenoic acid is effective for improving memory learning ability, improving visual acuity, suppressing inflammation, and reducing a plasma cholesterol level; docosapentaenoic acid is effective for prevention of arteriosclerosis and alleviation of symptoms of cancer or rheumatism; and eicosapentaenoic acid is effective for reduction in blood viscosity and increase in HDL. Thus, they are drawing much attention as promising materials for heath food.

These fatty acids have however the problem that owing to insufficient transfer to tissues, the intake of them does not bring about a satisfactory effect. With a view to overcoming this problem, studies have been made to promote systemic absorption of these fatty acids and a composition (for example, refer to Patent Document 1) having enhanced transfer of docosahexaenoic acid and/or eicosapentaenoic acid into the blood is one example of them.

These n-3 polyunsaturated fatty acids such as eicosapentaenoic acid and docosahexaenoic acid are often present in the form of triglyceride (fish oil) or glycerophospholipid (krill oil) in nature. It is reported that glycerophospholipid type ones show higher absorption than triglyceride type ones at the time of intake and in addition, they have high stability against deterioration due to oxidation so that they have recently been used as food materials (for example, refer to Patent Document 2 and Non-patent Document 1). Since such glycerophospholipid type polyunsaturated fatty acids exist as a solid or semi-solid at normal temperature, they cannot be used as are for food. Although they are used only in the form dissolved in linoleic acid, their systemic absorption or transfer to tissues is still insufficient. It has recently been elucidated that high intake of linoleic acid becomes a primary risk factor for heart diseases, cancers or allergic hypersensitivity so that oily compositions which are more useful and have higher safety are desired.

On the other hand, α-linolenic acid is contained in large amounts in linseed oil, perilla oil, egoma oil, and tung oil. It is a linear fatty acid having cis double bonds at 9, 12 and 15 positions. It is known to be effective for prevention of allergy, prevention of hypertension, prevention of mammary cancer, improvement of learning ability and improvement of visual acuity (for example, refer to Patent Document 3). Compositions comprising glycerophospholipid type docosahexaenoic acid and α-linolenic acid or a fat containing it however have not yet been known.
Patent Document 1: JP-A-2001-120189
Patent Document 2: JP-A-2001-186898
Patent Document 3: JP-A-Hei06-70717
Non-patent Document 1: Teruyuki Kaneda, and one other "Whether DNA contributes to longevity or not", Shokuhin to Kaihatsu, 34(8), 41-43(1999)

### DISCLOSURE OF THE INVENTION

### PROBLEMS THAT THE INVENTION IS TO SOLVE

An object of the present invention is to provide a composition permitting high systemic absorption of n-3 polyunsaturated fatty acids and high transfer of them to tissues and capable of fully exhibiting pharmacological effects which they have.

### MEANS FOR SOLVING THE PROBLEMS

With the foregoing in view, the present inventors have carried out an extensive investigation. As a result, it has been found that a fat composition prepared using, as a supply source of an n-3 polyunsaturated fatty acid, a phospholipid having the polyunsaturated fatty acid as a constituent fatty acid and incorporating therein α-linolenic acid and/or a fat containing α-linolenic acid enables drastic improvement in the systemic absorption of the polyunsaturated fatty acid, smooth transfer of it into tissues which require them, and sufficient exhibition of their pharmacological effects such as central nervous function improving effect and circulatory function improving effect.

In one aspect of the present invention, there is thus provided a fat composition comprising one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

In another aspect of the present invention, there is also provided a food or beverage comprising the above-described fat composition.

In a further aspect of the present invention, there is also provided a medicament comprising the above-described fat composition.

In a still further aspect of the present invention, there is also provided a central nervous function improving agent, which comprises one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

In a still further aspect of the present invention, there is also provided a circulatory function improving agent, which comprises one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

In a still further aspect of the present invention, there is also provided a method of improving a central nervous function, which comprises administering a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

In a still further aspect of the present invention, there is also provided a method of improving a circulatory function, which comprises administering a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

In a still further aspect of the present invention, there is also provided the use, for the preparation of a central nervous function improving agent, of a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

In a still further aspect of the present invention, there is also provided the use, for the preparation of a circulatory function improving agent, of a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

### ADVANTAGE OF THE INVENTION

Owing to high systemic absorption of n-3 polyunsaturated fatty acids, high transfer of them to tissues and sufficient exhibition of pharmacological effects which the fatty acids have, the fat composition of the present invention is useful as a food or beverage, or medicament effective for improving a central nervous function, visual acuity, circulatory function and the like.

### BEST MODE FOR CARRYING OUT THE INVENTION

The phospholipids in the present invention have, as a constituent fatty acid, an n-3 polyunsaturated fatty acid selected from docosahexaenoic acid, docosapentaenoic acid and eicosapentaenoic acid.
The term "the phospholipids have, as a constituent fatty acid, an n-3 polyunsaturated fatty acid" as used herein means that the phospholipids have an n-3 polyunsaturated fatty acid residue as an acyl group. The constituent fatty acids of each of the phospholipids may be either partially or wholly an n-3 polyunsaturated fatty acid.
Such phospholipids having, as a constituent fatty acid thereof, an n-3 polyunsaturated fatty acid may be any of those chemically synthesized, those extracted from natural products such as krill, squid and scallop, and those extracted from natural products and then subjected to chemical treatment or enzymatic treatment (JP-A-2001-186898, JP-A-9-121879, etc.). Examples of the phospholipids include phosphatidyl choline, phosphatidyl serine, phosphatidyl inositol, phosphatidyl ethanolamine, phosphatidic acid and phosphatidyl cardiolipin. Of these, phosphatidyl choline and phosphatidyl serine are preferred.
Especially, phosphatidyl choline having as a constituent fatty acid thereof docosahexaenoic acid(PC-DHA), phosphatidyl choline having as a constituent fatty acid thereof eicosapentaenoic acid(PC-EPA), phosphatidyl serine having as a constituent fatty acid thereof docosahexaenoic acid(PS-DHA), and phosphatidyl serine having as a constituent fatty acid thereof eicosapentaenoic acid(PS-EPA) are preferred.
In the fat composition of the present invention, these phospholipids may be used either singly or in combination of two or more.

The α-linolenic acid to be used in the present invention may be either chemically synthesized one or that obtained by extraction or purification of a natural product such as rapeseed, sunflower, soybean, linseed, perilla, egoma and tung.

Examples of the fat containing α-linolenic acid include rapeseed oil, sunflower oil, soybean oil, canola oil, linseed oil, perilla oil, egoma oil and tung oil. Two or more of these oils may be used as a mixture. Use of one or more of linseed oil, perilla oil, egoma oil and tung oil is preferred, with use of linseed oil being more preferred. In order to improve systemic absorption of docosahexaenoic acid, docosapentaenoic acid or eicosapentaenoic acid contained in the phospholipid, the above-described fat preferably contains from 0 to 0.7 part by weight, more preferably from 0 to 0.5 part by weight, still more preferably from 0 to 0.25 part by weight, still more preferably from 0 to 0.1 part by weight of linoleic acid relative to 1 part by weight of α-linolenic acid.
The fat containing no linoleic acid is especially preferred.

In the fat composition of the present invention, the α-linolenic acid or the α-linolenic-acid-containing fat is contained preferably in an amount of from 0.1 to 20 parts by weight, more preferably from 0.25 to 10 parts by weight, still more preferably from 0.5 to 4 parts by weight, especially preferably from 0.75 to 2 parts by weight, relative to 1 part by weight of the phospholipid.

The fat composition of the present invention may contain an oil extracted from fishes and shellfishes such as tuna, sardine, yellowtail, young yellowtail, mackerel, salmon caviar, krill and escallop, or an oil extracted from natural products or chemically synthesized oil such as docosahexaenoic acid, docosapentaenoic acid or eicosapentaenoic acid. Of these, preferred is an oil extracted from tuna, sardine or krill because it can be prepared easily.

The fat composition of the present invention may further contain a compound or crude drug for enhancing the central nervous function improving effect, visual acuity improving effect or circulatory function improving effect of the fat composition. Examples of the compound or crude drug for enhancing the central nervous function improving effect include araliaceous plants (ginseng, P. notoginseng and the like), Siberian ginseng, arginine, ginkgo biloba, soybean and carnitine; those of the compound or crude drug for enhancing the visual acuity include bilberry, blueberry, raspberry, rutin and cassis; and those of the compound or crude drug for enhancing the circulatory function improving effect include citric acid, sesame, sprouted sesame, rutin, garlic, onion, fermented soybeans, Echinacea, and black cohosh. These compounds or crude drugs may be used either singly or in combination of two or more. Of these, ginseng, P. notoginseng, Siberian ginseng, bilberry, arginine, ginkgo biloba and rutin are especially preferred crude drugs.

The fat composition of the present invention may further contain an ordinarily employed antioxidant. Examples of such an antioxidant include vitamin C, vitamin E, catechin, astaxanthin, sesamin, and polyphenols. They may be used either singly or in combination of two or more. Of these, vitamin E is preferred.

The fat composition of the present invention can be prepared by dissolving the phospholipid having, as a constituent fatty acid, an n-3 polyunsaturated fatty acid, the α-linolenic acid and/or the α-linolenic-acid-containing fat, and the above-described various components under stirring.

As described later in Examples, the fat composition of the present invention thus obtained is absorbed in the body and transferred into tissues at a higher rate than PS-DHA, PC-DHA or the like dissolved in linoleic acid and therefore exhibits excellent central nervous function improving effect, circulatory function improving effect and the like.
Accordingly, the fat composition of the present invention is useful as a medicament or a food or beverage exhibiting a central nervous function improving effect, circulatory function improving effect or the like. The food or beverage comprising the composition can be provided as an invalid diet or specified health food exhibiting a central nervous function improving effect, visual acuity improving effect or circulatory function improving effect. It can be provided as a food or beverage attached, at the product portion, package or catalogue thereof, with a label which indicates that it is a food or beverage for improving central nervous function, visual acuity or circulatory function.

For use as a food or beverage, the fat composition of the present invention can be provided in any beverage or food form such as solid food, gelled food, liquid food or the like, for example, liquid, powders, capsules, granules and tablets. Of these, capsules are preferred because the composition can readily be processed into capsules. Such food or beverage can be prepared in a conventional manner.

When the composition is provided as capsules, the filling in the capsule can be prepared using, in addition to the fat composition of the present invention, additives usually employed for foods, excipient, binder, disintegrant, stabilizer, dispersant, coloring agent, taste corrigent, plant oil and surfactant such as long chain unsaturated fatty acid, medium chain fatty acid glyceride, polyethylene glycol or glycerin fatty acid ester. Examples of the capsule form include soft capsules, hard capsules and microcapsules. Of these, soft capsules are preferred because they can be prepared readily. Capsules made of gelatin may preferably contain, in the filling thereof, soybean lecithin or egg yolk lecithin in order to prevent insolubilization.

The soft capsules are prepared, for example, by a stamping method using a rotary full-automatic soft capsule forming machine, a plate method of inserting the filling between two gelatin sheets, pressing dies on both sides against the sheets, and then stamping, and a drip method (seamless capsule) by using a double nozzle.

A medicament comprising the fat composition of the present invention may be prepared by adding a pharmaceutically acceptable carrier to the composition and following the conventional manner. No particular limitation is imposed on the form of the medicament and examples of it include capsules, tablets, pills, troches, powders and granules. Of these, capsules are preferred because they can be prepared easily.

The daily dosage (effective intake amount) of the medicament such as central nervous function improving agent or circulatory function improving agent or the food or beverage according to the present invention is preferably from 0.5 to 25 mg/kg weight in terms of DHA, especially preferably from 1 to 15 mg/kg weight, more preferably from 2 to 8 mg/kg weight.
The present invention will hereinafter be described by Examples. It should however be borne in mind that the present invention is not limited by them.

### EXAMPLES

### Example 1

Recently-weaned ICR female mice (three weeks old) were fed with a diet containing n-3 polyunsaturated fatty acids (about 3% α-linolenic acid/total lipids) or a diet deficient in n-3 polyunsaturated fatty acids (about 0.04% α-linolenic acid/total lipids), whereby normal mice and n-3 polyunsaturated fatty acid-deficient mice were obtained. When they became eight weeks old, they were crossed with nine-week-old ICR male mice to obtain the second-generation mice. Within two days after birth, the number of the second-generation mice thus obtained was reduced by culling to 10 per mother mouse. They were fed continuously with the same diet. A fat composition having PS-DHA (phosphatidyl serine having docosahexaenoic acid as a constituent fatty acid) dissolved in linoleic acid (safflower oil: containing linoleic acid and α-linolenic acid at a 780:1 ratio) and another fat composition having PS-DHA dissolved in α-linolenic acid (linseed oil: containing linoleic acid and α-linolenic acid at a 0.46:1 ratio) were prepared. Administration of these compositions was started when the mice of the second generation became 7 weeks old. A daily administration amount was 6 mg in terms of DHA and 9 mg in terms of PS and the DHA levels in the serum and in the brain were measured one week after the administration was started.
The results are shown in Table 1.

**[Table 1] DHA content (%) in all fatty acids: n=5**

| Test group | PS-DHA + linoleic acid (safflower oil) | PS-DHA + α-linolenic acid (linseed oil) |
|---|---|---|
| Serum | 4.24 ± 0.10 | 6.60 ± 0.23 |
| Brain | 5.20 ± 0.17 | 6.03 ± 0.12 |

From Table 1, it has been understood that a DHA content in each of the serum and brain was higher in the group in which the phospholipid having DHA bound thereto as an acyl group was dissolved in α-linolenic acid than in the group of linoleic acid. This suggests that when DHA was dissolved in α-linolenic acid, DHA was absorbed rapidly into the DHA-deficient body and transferred into its tissues. The final DHA content in all the fatty acids in the brain tissues becomes about 16% so it is presumed that very good effects for the central nervous function are produced when the DHA content approaches this percentage as soon as possible.

### Example 2

Recently-weaned ICR female mice (three weeks old) were fed with a diet containing an n-3 polyunsaturated fatty acid (about 3% α-linolenic acid/total lipids) or a diet deficient in an n-3 polyunsaturated fatty acid (about 0.04% α-linolenic acid/total lipids), whereby normal mice and n-3 polyunsaturated fatty acid-deficient mice were obtained. When they became eight weeks old, they were crossed with nine-week-old ICR male mice to obtain mice of the second generation. Within two days after birth, the number of the second-generation mice thus obtained was reduced by culling to 10 per mother mouse. They were fed continuously with the same diet. When the second generation mice became 7 weeks old, administration of the test drugs prepared in Example 1 was started. Administration was performed orally once a day. Step-through type learning test was conducted for 11 days from 2.5 weeks to 4 weeks after administration was started.
The results are shown in Table 2.

### (Step-through type learning test)

A test cage ("Model PA-M1", product of Obara Ika Sangyo) having a dark compartment in which electric shock can be given and a light compartment was employed. Mice have a habit of moving to the dark compartment when placed in the light compartment. Electric shock of about 36V is given to the grids of the floor of the dark compartment so mice return to the light compartment quickly in order to avoid the electric shock. The mouse once electrically stimulated was returned to a home cage in order to receive no more stimulation in this session. Similar operation was repeated for 10 days while setting the measurement time at 5 minutes at maximum and each group was evaluated using frequency of failure for 10 days as an index.

**[Table 2] Frequency (times) of failure for 10 days in n-3 polyunsaturated fatty acid-deficient mice**

| PS-DHA + linoleic acid (safflower oil) | PS-DHA + α-linolenic acid (linseed oil) |
|---|---|
| 1.00 ± 0.26 | 0.86 ± 0.14 |

From Table 2, it has been found that the frequency of failure for the test period of 10 days is smaller in the group for which linseed oil containing a large amount of α-linolenic acid was administered than in the group for which safflower oil containing a large amount of linoleic acid was administered. Even if a difference in learning effect is slight, repetition of learning synergistically increases the difference. The difference found from Table 2 therefore proves the usefulness of α-linolenic acid sufficiently.

### Example 3: Example of PC-DHA

In a similar manner to Example 1 and Example 2, ICR mice, that is, the second generation of n-3 polyunsaturated fatty acid-deficient mice were obtained. A fat composition obtained by dissolving PC-DHA in linoleic acid and another fat composition obtained by dissolving PC-DHA in α-linolenic acid were each administered in an amount of 11 mg in terms of DHA and 11 mg in terms of PC to the male mice of the second generation which became from 10 to 13 weeks old. The DHA level in the plasma two hours after the administration, and the DHA level in the plasma and erythrocyte on Day five after administration for four straight days were measured. The results are shown in Table 3.

**[Table 3] DHA content (%) in all fatty acids: n=5**

| Test group | PC-DHA + linoleic acid (safflower oil) | PC-DHA + α-linolenic acid (linseed oil) |
|---|---|---|
| Plasma after 2 hours | 2.42 ± 0.13 | 3.43 ± 0.40 |
| Plasma on Day 5 | 6.03 ± 0.56 | 7.22 ± 0.25 |
| Erythrocyte on Day 5 | 2.37 ± 0.09 | 2.78 ± 0.20 |

From Table 3, it has been found that similar to Example 1, when PC-DHA is dissolved in α-linolenic acid, DHA is absorbed rapidly in the body deficient in DHA and transferred to tissues. PC-DHA is known to specifically transfer to erythrocytes. The final DHA content in all the fatty acids in the erythrocyte is about 6%, so it is presumed that when the DHA content approaches this percentage as soon as possible, very good effects for the circulatory function, for example, increase in fluidity of erythrocytes are produced.

Example 4: Preparation of soft capsules (1) Components shown in Table 4 were stirred in a homogenizer (at 1000 rpm for 5 minutes) to prepare a filling.

**[Table 4]**

| Components | A-1 (%) | A-2 (%) |
|---|---|---|
| Linseed oil | 50 | 50 |
| PC-DHA | 40 | - |
| PS-DHA | - | 40 |
| Soybean lecithin | 10 | 10 |

(2) A gelatin solution composed of 45 wt.% of gelatin, 15 wt.% of glycerin and 40 wt.% of water was dissolved at 80°C, followed by defoaming. The resulting solution was allowed to stand for about 10 hours. The fillings (A-1 and A-2), each 360 mg, prepared above in (1) was filled using a rotary type soft capsule filling machine ("Oval 6 type"). The capsules filled with the respective fillings were dried at 27°C and humidity of 50% or less until the water content in the film decreased to 8%, whereby soft capsules were prepared. No change in the filling occurred even after 1 week storage at 40°C and humidity of 75%. In addition, no problem occurred in the disintegration property of the capsules.

## Claims

1. A fat composition comprising one or more phospholipids having, as a constituent fatty acid, an n-3 polyunsaturated fatty acid selected from docosahexaenoic acid, docosapentaenoic acid and eicosapentaenoic acid; and α-linolenic acid and/or a fat containing α-linolenic acid.

2. The fat composition according to Claim 1, wherein the phospholipid is phosphatidyl choline, phosphatidyl serine, phosphatidyl ethanolamine, phosphatidyl inositol, phosphatidic acid or phosphatidyl cardiolipin.

3. The fat composition according to Claim 1 or 2, wherein the fat containing α-linolenic acid is derived from a plant selected from linseed, perilla, egoma or tung.

4. The fat composition according to any one of Claims 1 to 3, further comprising an oil extracted from fishes and fisheries.

5. The fat composition according to any one of Claims 1 to 4, further comprising one or more compounds or crude drugs having a central nervous function improving effect, visual acuity improving effect or circulatory function improving effect.

6. The fat composition according to Claim 5, wherein the compounds or crude drugs are selected from ginseng, P. notoginseng, Siberian ginseng, bilberry, arginine, ginkgo biloba, and rutin.

7. A food or beverage comprising a fat composition as claimed in any one of Claims 1 to 6.

8. The food or beverage according to Claim 7, which is attached with a label indicating that the food or beverage is used for improving central nervous function, visual acuity or circulatory function.

9. A medicament comprising a fat composition as claimed in any one of Claims 1 to 6.

10. An agent for improving central nervous function, which comprises one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

11. An agent for improving circulatory function, which comprises one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

12. A method for improving central nervous function, which comprises administering a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

13. A method for improving circulatory function, which comprises administering a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

14. Use, for the preparation of an agent for improving central nervous function, of a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.

15. Use, for the preparation of an agent for improving circulatory function, of a fat composition containing one or more phospholipids having, as a constituent fatty acid, an n-3 polyunsaturated fatty acid selected from docosahexaenoic acid, docosapentaenoic acid and eicosapentaenoic acid, and α-linolenic acid and/or a fat containing α-linolenic acid.
